# EUROPEAN PATENT SPECIFICATION

(11) **EP 1 675 621 B1**
(45) Date of publication and mention of the grant of the patent: **28.02.2007**
(21) Application number: 04764259.0
(22) Date of filing: 18.08.2004
(51) Int. Cl.: A61K 47/44, A61K 47/14, A61K 47/10, A61K 47/06, A61K 31/195

(54) **PHARMACEUTICAL COMPOSITIONS OF LAVENDUSTIN**
PHARMAZEUTISCHE ZUSAMMENSETZUNGEN VON LAVENDUSTIN
COMPOSITIONS PHARMACEUTIQUES DE LAVENDUSTINE

(30) Priority: 19.08.2003 GB 0319497
(43) Date of publication of application: 05.07.2006
(73) Proprietor: Novartis AG, 4056 Basel (CH); Novartis Pharma GmbH, 1230 Wien (AT)
(72) Inventor: KRIWET, Katrin, 79639 Grenzach-Wyhlen (DE)
(74) Representative: Leon, Susanna Iris
(86) International application number: PCT/EP2004/009273
(87) International publication number: WO 2005/016322

(56) References cited:
- WO-A-96/28430
- DATABASE WPI Section Ch, Week 199504 Derwent Publications Ltd., London, GB; Class B05, AN 1995-027620 XP002335322 & JP 06 312919 A (KAO CORP) 8 November 1994 (1994-11-08)
- MOSER K ET AL: "Enhanced skin permeation of a lipophilic drug using supersaturated formulations" JOURNAL OF CONTROLLED RELEASE, ELSEVIER SCIENCE PUBLISHERS B.V. AMSTERDAM, NL, vol. 73, no. 2-3, 15 June 2001 (2001-06-15), pages 245-253, XP004246465 ISSN: 0168-3659

## Description

The invention relates to topical pharmaceutical compositions in the form of an emulsion comprising a lavendustin derivative.

It particularly concerns **topical pharmaceutical compositions** comprising **a lavendustin derivative of formula I** 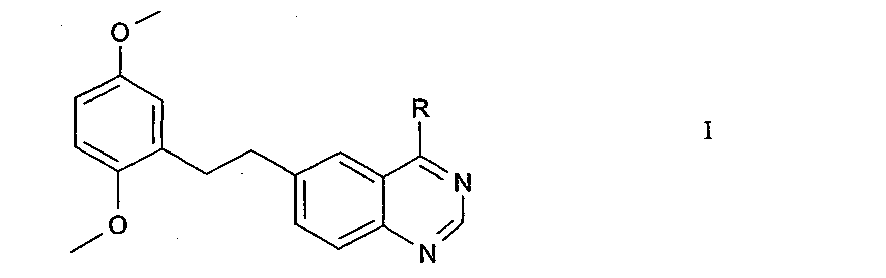 wherein R is methyl, methoxy or ethyl, or a pharmaceutically acceptable salt thereof, and **an emollient,**
hereinafter briefly named "**the compositions of the invention**".

The compounds of formula I are known, e.g. as Example 6 (R = ethyl, hereinafter **compound A**), Example 16 (R = methoxy, hereinafter **compound B**), and Example 17 (R = methyl, hereinafter **compound C**) in USP 5'990'116, the contents of which is incorporated herein by reference.

Preferred is 6-[2-(2,5-dimethoxyphenyl)ethyl]-4-ethyl-quinazoline (compound A).

These compounds are useful in the topical treatment of hyperproliferative disorders such as actinic keratosis, anogenital warts and seborrhoic keratosis, and skin cancer. Hyperproliferative skin disorders are often accompanied by a hyperkeratosis. In particular, actinic keratosis is a skin disease with horny and dry skin lesions.

Treatment of hyperproliferative disorders can include destructive methods (cryotherapy, electro-desiccation and curettage, excisional therapy) and topical chemotherapy. Destructive methods and surgery may cause pain, blistering, scars and pigment changes and therefore, especially for patients with multiple lesions, topical chemotherapy is preferred.

Little is known about the mechanism of action of lavendustins, e.g. of formula I as defined above, but like other antiproliferative agents they may cause skin irritation. For example, a composition of 0.5 % w/w of lavendustin of formula (I) in ethanol/water showed severe skin irritation potential in an in vitro human epidermis model.

It has now been found that compositions comprising lavendustin or a lavendustin derivative, such as those of formula I as defined above and an emollient may be formulated into compositions of good physico-chemical stability, having good penetration and good tolerability and allowing application on skin, e.g. face, and mucous membranes. They avoid high local concentration of the lavendustin in the skin and mucous membrane and therefore are well tolerated.

Accordingly the invention provides in one aspect, a **topical pharmaceutical composition comprising lavendustin or a lavendustin derivative**, e.g. of formula I as defined above or a pharmaceutically acceptable salt thereof, **and an emollient**.

Suitable emollients may be selected from e.g.:
i) liquid **fatty alcohols**, saturated and/or unsaturated, branched and/or unbranched, having e.g. a C₈ to C₂₄ chain. Preferred is **oleyl alcohol**, e.g. as known and commercially available under the trademark **HD Eutanol^{R}** from e.g. Henkel, Germany;
ii) liquid **waxes**, e.g. natural, synthetic, semisynthetic or emulsifying waxes, preferably **isopropyl myristate**, e.g. as known and commercially available from Henkel, Germany; oleyl erucate, e.g. as known and commercially available under the trademark Cetiol^{R} J600 from e.g. Henkel, Germany; diisopropyl adipate, e.g. as known and commercially available under the trademark Isopat® 1794 from e.g. Dargoco, Germany; and/or oleyl oleate, e.g. as known and commercially available under the trademark Cetiol^{R} from e.g. Henkel, Germany;
iii) **di- and triglycerides**, having e.g. C₈ to C₂₄ fatty acids, e.g. a **medium chain fatty acid triglyceride**, e.g. **Miglyol^{R} 812**. Miglyol^{R} 812 is a fractionated coconut oil comprising caprylic-capric acid triglycerides and having a molecular weight of about 520 daltons. Fatty acid composition: C₆ max. about 3 %, C₈ about 50 to 65 %, C₁₀ about 30 to 45 %, C₁₂ max. 5 %; acid value about 0.1; saponification value about 330 to 345; iodine value max. 1. Miglyol^{R} 812 is commercially available from e.g. Hüls Chemie AG, Germany;
iv) **propylene glycol mono- and di-fatty acid esters** such as propylene glycol caprylate, commercially available under the trademark Miglyol^{R} 840 (H.P. Fiedler, Lexikon der Hilfsstoffe für Pharmazie, Kosmetik und angrenzende Gebiete 2 [2002] 5th Ed., Editio Cantor Verlag Aulendorf, p. 1130-1131), propylene glycol dilaurate, propylene glycol hydroxystearate, propylene glycol isostearate, propylene glycol laurate, propylene glycol ricinoleate, and propylene glycol stearate;
v) **petrolatum, e.g. white petrolatum**, e.g. as known and commercially available from e.g. Mineral Chemie AG, Germany; and
vi) mixtures of any of components i) to v).

The lavendustin or lavendustin derivative is present in the compositions of the invention in an amount of from about 0.01 to about 10 %, e.g. from about 0.05 to about 3 %, e.g. from about 0.1 to about 2 %, e.g. from about 0.2 to about 1 %, e.g. about 0.2 or about 0.8 % by weight based on the total weight of the composition.

The emollient may be present in an amount of from about 5 to about 40 %, preferably from about 5 to about 30 % by weight based on the total weight of the composition.

The compositions of the invention are preferably in form of emulsions, more preferably in the form of oil-in-water emulsions. Accordingly, the invention further provides topical **pharmaceutical compositions in form of an emulsion**, e.g. in form of an oil-in-water emulsion, **comprising lavendustin or a lavendustin derivative**, e.g. of formula I, or a pharmaceutically acceptable salt thereof, **and an emollient**.

Preferably, the lavendustin or lavendustin derivative, e.g. of formula I, or a pharmaceutically acceptable salt thereof is dissolved in the lipophilic components, such as the emollient(s), and is released from the formulation in a uniform and sustained manner thereby avoiding high local concentrations, i.e. concentrations which could cause irritation, of the lavendustin derivative in the skin or mucous membrane.

In another aspect, the invention thus provides **topical pharmaceutical compositions comprising lavendustin or a lavendustin derivative**, e.g. of formula (I), or a pharmaceutically acceptable salt thereof **which avoid high local concentration** of the lavendustin in the skin or mucous membrane and therefore are well tolerated.

The compositions of the invention may further comprise **hydrophilic components** such as **propylene glycol**; hexylene glycol; liquid polyethylene glycol such as PEG 200, 300, 400 or 600; and/or **glycerol (glycerine)**. The hydrophilic components may be present in amounts of from about 1 up to about 20 %, e.g. from about 1 to about 5 % by weight based on the total weight of the composition.

The compositions of the invention may further comprise **water**, e.g. **purified water**. Water may be present in amounts of from about 20 up to about 90, e.g. from about 35 to about 80 % by weight based on the total weight of the composition.

The compositions of the invention may further comprise **emulsifiers**. Such emulsifiers are described in standard texts such as Fiedler, loc. cit. ; and A.H. Kibbe Handbook of Pharmaceutical Excipients (2000), a joint publication of Pharmaceutical Press, London (UK) and American Pharmaceutical Association, Washington (US). Examples of suitable emulsifiers include:
i) **sorbitan fatty acid esters,** e.g. sorbitan mono C₁₂₋₁₈ fatty acid esters; sorbitan sesqui C₁₂₋₁₈ fatty acid esters; and sorbitan tri C₁₂₋₁₈ fatty acid esters, as known and commercially available under the trademark Span® or Arlacel®. Particularly preferred are the products
   - Span^{R} 20, a sorbitan monolaurate, having a D²⁵ of about 1, a HLB of about 8.6, and a viscosity of about 3900 to 4900 mPa's;
   - **Arlacel^{R} 83, a sorbitan monosesquioleate,** having a D²⁵ of about 1, a HLB of about 3.7, and a viscosity of about 1500 mPa's; and
   - **Span^{R} 60, a sorbitan monostearate,** having a HLB of about 4.7, and an acid value of about 5 to 10 (Fiedler, loc. cit., p. 1571-1572; Handbook of Pharmaceutical Excipients, loc. cit., page 511);
ii) polyoxyethylene-sorbitan-fatty acid esters, for example mono- and tri-lauryl, palmityl, stearyl and oleyl esters of the type also called polysorbates, known and commercially available under the trademark Tween® (Fiedler, loc. cit. p. 1754-1757; Handbook of Pharmaceutical Excipients, loc. cit., p. 416), including the products **Tween^{R}**
   - **20 [polyoxyethylene(20)sorbitanmonolaurate]**;
   - 21 [polyoxyethylene(4)sorbitanmonolaurate];
   - 40 [polyoxyethylene(20)sorbitanmonopalmitate];
   - **60 [polyoxyethylene(20)sorbitanmonostearate];**
   - 65 [polyoxyethylene(20)sorbitantristearate];
   - 80 [polyoxyethylene(20)sorbitanmonooleate];
   - 81 [polyoxyethylene(5)sorbitanmonooleate];
   - 85 [polyoxyethylene(20)sorbitantrioleate].
   Especially preferred products of this class are Tween^{R} 20 and Tween^{R} 60;
iii) **salts of fatty alcohol sulfates such as sodium lauryl sulfate and sodium cetylstearyl sulfate**, preferably sodium cetylstearyl sulfate as known and commercially available under the trademark **Lanette^{R} E** (Fiedler, loc. cit.. p. 1007-1008) from Henkel, Germany;
iv) **polyoxyethylene alkyl ethers**, e.g. polyoxyethylene glycol ethers of C₁₂ to C₁₈ alcohols, e.g. polyoxyethylene cetyl ether or polyoxyethylene oleyl ether, or polyoxyethylene stearyl ether, as known and commercially available under the trademark Brij^{R} (Fiedler, loc. cit., p. 325-327; Handbook of Pharmaceutical Excipients, loc. cit., page 407);
v) **polyoxyethylene fatty acid esters**, for example polyoxyethylene stearic acid esters of the type known and commercially available under the trademark Mytj^{R} (Fiedler, loc. cit., p. 1166-1167; Handbook of Pharmaceutical Excipients, loc. cit., page 420). An especially preferred product of this class is e.g. Myrj^{R} 52, a polyoxyethylene 40 stearate having D²⁵ of about 1.1, a melting point of about 40 to 44°C, a HLB value of about 16.9, an acid value of about 0 to 1 and a saponification number of about 25 to 35;
vi) **polyoxyethylene-polyoxypropylene copolymers and block co-polymers** such as those known and commercially available under the trade names Pluronic^{R}, Emkalyx^{R}, and Poloxamer^{R} (Fiedler, loc. cit., p. 1330-1332, 1334; Handbook of Pharmaceutical Excipients, loc. cit. page 386) and in particular Poloxamer^{R} 188 and Pluronic^{R} F68 having a D²⁵ of about 1.1, a melting point of about 40 to 44°C, and a HLB value of about 16.9;
vii) **esters of polyethylene-glycol glycerol ethers which have at least one free hydroxyl group and aliphatic C₆-C₂₂ carboxylic acids**. Examples include **glycerine mono stearate (PEG-20)**;
viii) **reaction products of a natural or hydrogenated castor oil and ethylene oxide**. The polyethyleneglycol hydrogenated castor oils are available under the trademark Cremophor^{R} (Fiedler, loc. cit.. p. 472-475). Particularly suitable are:
   - Cremophor^{R} RH 40, having a saponification value of about 50 to 60, an acid value of less than about 1, a water content (Fischer) of less than about 2 %, a n_{D}⁶⁰ of about 1.453 to 1.457 and a HLB of about 14 to 16;
   - Cremophor^{R} RH 60, having a saponification value of about 40 to 50, an acid value of less than about 1, an iodine value of less than about 1, a water content (Fischer) of about 4.5 to 5.5%, a n_{D}²⁵ of about 1.453 to 1.457 and a HLB of about 15 to 17; and
   - Cremophor^{R} EL, having a molecular weight (by steam osmometry) of about 1630, a saponification value of about 65 to 70, an acid value of about 2, an iodine value of about 28 to 32 and a n_{D} ²⁵ of about 1.471.
      Also suitable are various tensides available under the trademark Nikkol^{R} (Fiedler, loc. cit.. p. 1210-1211), Emulgin^{R} (Fiedler, loc. cit., p. 637), Mapeg^{R} (Fiedler, loc. cit. p. 1086) and Incrocas^{R} (Fiedler, loc. cit.. p. 908);
ix) **polyethylene glyceride** as commercially available under the trademark Labrafil^{R} (Fiedler, loc. cit., p. 880), particularly Labrafil^{R} M2130 CS;
x) **glycerine sorbitan fatty acid esters** as commercially available under the trademark Arlacel^{R} 481, having a molecular weight of about 630, and a HLB value of about 4.5 (Fiedler, loc. cit., p. 247-249); and
xi) mixtures of any of components i) to x).

It is to be appreciated that emulsifiers may be complex mixtures containing side products or unreacted starting products involved in the preparation thereof, e.g. emulsifiers made by polyoxyethylation may contain another side product, e.g. polyethylene glycol.

When the composition is a water-in-oil emulsion, the emulsifier selected preferably has a HLB value of about 10 to 15. When the emulsion is an oil-in-water emulsion, the emulsifier selected preferably has a HLB value of about 4 to 8. A combination of emulsifiers having different HLB values may be used to achieve a desired HLB value. Preferably the emulsifiers are present in an amount of from about 1 to about 30 % by weight based on the total weight of the composition, more preferably from about 10 to about 25 %.

Preferably, the compositions of the invention are in form of **a cream, a lotion** or **an emulsion gel**, especially a cream.

If desired, the compositions of the invention may comprise **consistency agents**, preferably a mixture of consistency agents. Suitable consistency agents include e.g.:
i) **solid alcohols,** having e.g. a C₁₂ to C₂₄ chain, e.g. cetyl **alcohol** and/or **stearyl alcohol**. Cetyl alcohol and stearyl alcohol may be commercially available e.g. under the trademarks **Lorol^{R} C16** and **Lorol^{R} C18**, respectively, from Henkel, Germany;
ii) **esters of fatty acid and fatty alcohols**. They may include esterified compounds of fatty acid having e.g. a C₁₂ to C₂₄ chain, saturated or unsaturated, and primary alcohol having e.g. a C₁₂ to C₂₄ chain, e.g. cetyl palmitate as commercially available under the trademark **Cutina^{R} CP** from Henkel, Germany;
iii) **glycerine monostearate** known and commercially available under the trademark Imwitor^{R} (Fiedler, loc. cit., p. 906-907; Handbook of Pharmaceutical Excipients, loc. cit. page 225), particularly Imwitor^{R} 960;
iv) **solid fatty acids**, having e.g. a C₁₂ to C₂₄ chain, e.g. stearic acid and its salts, e.g. aluminium stearate or magnesium stearate;
v) **solid waxes**, e.g. bees wax or carnauba wax; and
vi) mixtures of any of components i) to v).

Consistency agents are preferably present in an amount of from about 1 to about 30 %, e.g. from about 4 to about 10 % by weight based on the total weight of the composition.

If desired, the compositions of the invention may comprise **gelling agents**. Suitable **gelling agents** include carbomers, e.g. crosslinked poly(acrylic acid) polymers such as known and commercially available under the trademark Carbopol^{R} (Fiedler, loc. cit., p. 367-372; Handbook of Pharmaceutical Excipients, loc. cit., page 79). Carbopol^{R} 974P and Carpopol^{R} 1342 are preferred. The gelling agents are preferably present in an amount of from about 0.2 to about 2 %, more preferably less than about 1 % by weight based on the total weight of the composition.

The compositions of the invention may further comprise **preserving agents**, e.g. microorganism growth inhibitors such as methyl- or propylparabene, phenyl alcohol, **benzyl alcohol, propylene glycol**, sorbic acid, and chlorocresol as appropriate. Preserving agents are preferably present in an amount of from about 0.05 to about 1 % by weight based on the total weight of the composition.

The compositions may further comprise **antioxidants** such as butyl-hydroxytoluene, ascorbyl palmitate, sodium pyrosulfite, butyl-hydroxy anisole, propyl p-hydroxybenzoate, methyl p-hydroxybenzoate and tocopherol, as appropriate. Antioxidants are preferably present in an amount of from about 0.01 to about 2.5 % by weight based on the total weight of the composition.

If desired, **pH modifying agents** may be included to bring the pH of the composition to e.g. between about 4 and 6, or by adding a pharmaceutically acceptable buffer system, or by addition of solution (10 %) of sodium hydroxide. A pH of between 4 and 6 is desirable to avoid skin and mucous membrane irritation.

It will be appreciated that although the excipients have been described above by reference to a particular function, any particular excipient may have alternative or multiple functions, e.g. propylene glycol may act as e.g. hydrophilic component and/or preserving agent.

The compositions of the invention may be prepared in conventional manner by dissolving the appropriate amount of lavendustin in the lipophilic components such as lipophilic carrier and consistency agent at elevated temperature, e.g. at from about 60 to about 80 °C, and adding the aqueous phase under stirring and homogenization. Further hydrophilic excipients like buffering agents, gelling agents and preservatives are preferably added to the water phase. Emulsifiers may be added either to the lipophilic or hydrophilic components depending on their HLB values. After homogenization the resultant composition is cooled to room temperature under stirring.

Accordingly, in another aspect the invention provides a **process** for the preparation of a composition of the invention, comprising dissolving a lavendustin derivative of formula I as defined above, or a pharmaceutically acceptable salt thereof, in emollient and optionally further lipophilic components such as consistency agents, if present, preferably at elevated temperature, e.g. at from about 60 to about 80 °C, and adding the water phase, if present, under stirring and homogenization.

The compositions of the invention are indicated for use in the known indications of lavendustin, particularly in the treatment of hyperproliferative disorders such as actinic keratosis, anogenital warts and seborrhoic keratosis, and skin cancer.

Therefore, in a further aspect the invention provides a composition of the invention **for use in the treatment of hyperproliferative disorders** such as actinic keratosis, anogenital warts and seborrhoic keratosis, and skin cancer.

In another aspect the invention provides a **method for treating hyperproliferative** disorders such as actinic keratosis, anogenital warts and seborrhoic keratosis, and skin cancer comprising administering a composition of the invention to the skin or mucous membrane of a patient in need thereof.

In yet another aspect the invention provides the **use** of a composition of the invention **in the preparation of a medicament** for the treatment of hyperproliferative disorders such as actinic keratosis, anogenital warts and seborrhoic keratosis, and skin cancer.

The utility of the compositions of the invention may be observed in standard clinical tests.

A representative clinical trial may be carried out as follows:
A randomised single-centre, double-blind, within-subject vehicle-controlled study of a composition of the invention at a dose of 0.1 to 2 % active agent by weight based on the total weight of the composition over e.g. 10 cm², corresponding to a dose of about 0.1 to 1 mg/cm², is performed in subjects with actinic keratosis, anogenital warts and/or seborrhoic keratosis, and/or skin cancer. In total 15 to 36 subjects are treated with the composition once or twice daily for up to two weeks. The therapeutic effect on erythema, edema, pruritus, burning/stinging/pain, and erosion is evaluated separately for each treated lesion. Local tolerability and cosmetic outcome of each treatment and routine safety parameters, including hematology and blood chemistry, are assessed. The compositions of the invention are found to be effective.

The exact amount of lavendustin and composition to be administered depends on several factors, for example the desired duration of treatment and the rate of release of the active agent. Satisfactory results are obtained in larger subjects, e.g. humans, with local application over the area to be treated of a 0.01 to 10 % by weight concentration based on the total weight of the composition, e.g. 0.05 to 3%, preferably 0.1 to 2%, more preferably 0.2 to 1 %, most preferably about 0.8%, of the lavendustin once or several times a day (for example 1 to 5 times a day). In general, the compositions may be applied to areas of skin and mucous membranes as small as 1 cm² to as large as 1 m². Suitable skin and mucous membrane loadings fall within the range of 0.1 mg/cm² to 10 mg/cm², e.g. 2 mg/cm², of lavendustin composition.

The following Examples illustrate the invention. The following abbreviations are used:
Ex. = Example No.
v = volume

### Examples 1 to 9: Oil-in-water cream emulsions

| Component [in g] | **Ex.1** | **Ex.2** | **Ex.3** | **Ex.4** | **Ex.5** | **Ex.6** | **Ex.7** | **Ex.8** | **Ex.9** |
|---|---|---|---|---|---|---|---|---|---|
| Active ingredient: | | | | | | | | | |
| **Compound A** | 3 | 1 | 0.8 | 1 | 0.8 | 0.5 | 0.2 | 0.6 | 1.0 |

| Emollient: | | | | | | | | | |
|---|---|---|---|---|---|---|---|---|---|
| **i) oleyl alcohol** (HD Eutanol^{R}) | 10 | - | - | - | - | - | - | - | - |
| ii) **isopropyl myristate** (Henkel) | - | 10 | 10 | 9 | 12 | - | 8.0 | 8.0 | 8.0 |
| iii) **medium chain fatty acid triglyceride** (Miglyol^{R} 812) | 14 | - | - | - | - | 7.5 | - | - | - |
| v) **petrolatum white** | - | - | 4 | 15 | - | - | 25 | - | - |

| Hydrophilic component: | | | | | | | | | |
|---|---|---|---|---|---|---|---|---|---|
| propylene glycol (also as preserving agent) | 5 | 5 | - | - | 5 | 10 | - | - | - |
| glycerine (glycerol) | - | - | 5 | - | - | - | - | - | - |

| Emulsifier: | | | | | | | | | |
|---|---|---|---|---|---|---|---|---|---|
| i) sorbitan monostearate (Span 60^{R}) | - | - | - | 1.9 | 1.9 | - | 2.0 | 2.0 | 2.0 |
| i) sorbitan mono sesqiooleate (Arlacel^{R} 83) | - | 1 | - | - | - | - | - | - | - |
| ii) polyoxyethylene (20)sorbitanmonolaurate (Tween^{R} 20) | - | - | 2 | - | - | - | - | - | - |
| ii) polyoxyethylene (20)sorbitanmonostearate (Tween^{R} 60) | - | 3 | - | 6.1 | 6.1 | 7 | 6.0 | 6.0 | 6.0 |
| iii) sodium cetyl stearyl sulfate (Lanette^{R} E) | 1 | - | - | - | - | - | - | - | - |
| vii) glycerol monostearate (PEG-20) | 2 | 2 | - | - | - | 4 | - | - | - |

| Consistency agent: | | | | | | | | | |
|---|---|---|---|---|---|---|---|---|---|
| i) cetyl alcohol (Lorol^{R} C 16) | 4 | - | 5 | 4 | 4 | 6 | 4.0 | 4.0 | 4.0 |
| i) stearyl alcohol (Lorol^{R} C18) | 4 | 12 | 5 | 4 | 4 | - | 4.0 | 4.0 | 4.0 |
| ii) cetyl palmitate (Cutina^{R} CP) | - | 3 | - | - | 2 | - | 2.0 | 2.0 | 2.0 |

| Preserving agent: | | | | | | | | | |
|---|---|---|---|---|---|---|---|---|---|
| benzyl alcohol | - | - | - | - | 1 | - | 1.0 | 1.0 | 1.0 |
| Water, purified | to 100 | to 100 | to 100 | to 100 | to 100 | to 100 | to 100 | to 100 | to 100 |

The formulations of Examples 1 to 9 show good physico-chemical stability, and are mild to moderate irritants as determined in vitro using a human epidermis model, and also in a clinical study (see Example 11).

In Examples 1 to 9 compound A may be replaced with compound B or compound C.

### Example 10: In vitro penetration

*In vitro* penetration of compound A of Examples I to 4 into human skin was good; concentration of compound A found after 8 hours in the epidermis was between 0.4 and 1.3 µg/cm² (calf serum / PBS 1:2 v/v; pH = 7.4).

### Example 11: Clinical study

In total 36 subjects with actinic keratosis were treated once or twice daily over up to two weeks with the composition of Example 4. The formulation of Example 4 was effective. Local tolerability and cosmetic outcome were good. Adverse events were minor. Plasma levels of compound A were low, ranging from 0.034 to 3.3 ng/ml.

## Claims

1. A topical pharmaceutical composition comprising a lavendustin derivative of formula I 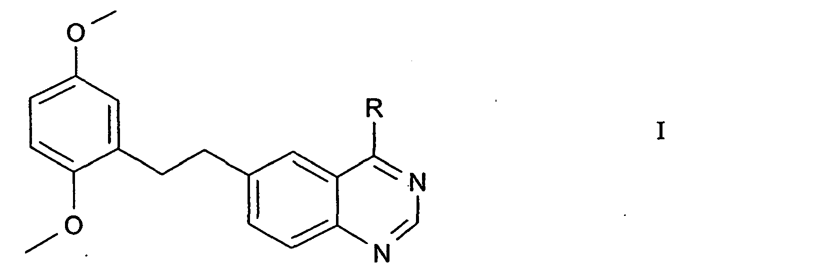 wherein R is methyl, methoxy or ethyl, or a pharmaceutically acceptable salt thereof, and an emollient.

2. A composition according to claim 1 wherein the emollient isopropyl myristate.

3. A composition according to claim 1 in the form of an emulsion.

4. A composition according to claim 3 in the form of an oil-in-water emulsion.

5. A topical pharmaceutical composition according to any one of claims 1 to 4 which avoids high local concentration of the lavendustin in the skin or mucous membrane and is well tolerated.

6. A process for the preparation of a composition according to claim 1, comprising dissolving a lavendustin derivative of formula I as defined in claim 1, or a pharmaceutically acceptable salt thereof, in emollient and optionally further lipophilic components such as consistency agents, if present, preferably at elevated temperature, e.g. at from about 60 to about 80 °C, and adding the water phase, if present, under stirring and homogenization.

7. A composition according to any one of claims 1 to 4 for use in the treatment of hyperproliferative disorders such as actinic keratosis, anogenital warts and seborrhoic keratosis, and skin cancer.

8. Use of a composition according to any one of claims 1 to 4 in the preparation of a medicament for the treatment of hyperproliferative disorders such as actinic keratosis, anogenital warts and seborrhoic keratosis, and skin cancer.

9. Use according to claim 8 wherein a composition according to any one of claims 1 to 4 is to be administered to the skin or mucous membrane of a patient in need thereof.

## Patentansprüche

1. Topische pharmazeutische Zusammensetzung, umfassend ein Lavendustinderivat der Formel I 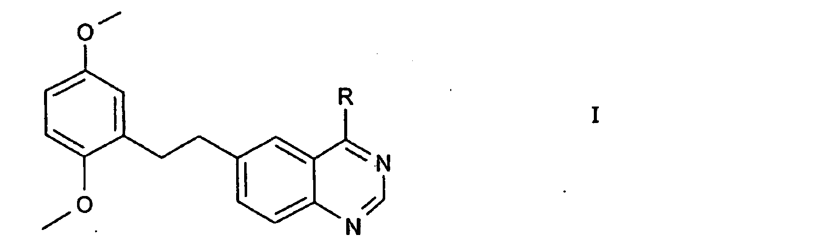 worin R für Methyl, Methoxy oder Ethyl steht, oder ein pharmazeutisch akzeptables Salz hiervon, und ein Emolliens.

2. Zusammensetzung nach Anspruch 1, worin das Emolliens Isopropylmyristat ist.

3. Verfahren nach Anspruch 1 in Form einer Emulsion.

4. Verfahren nach Anspruch 3 in Form einer Öl-in-Wasser-Emulsion.

5. Topische pharmazeutische Zusammensetzung nach einem der Ansprüche 1 bis 4, die eine hohe lokale Konzentration des Lavendustins in der Haut oder der Mukosa vermeidet und gut toleriert wird.

6. Verfahren zur Herstellung einer Zusammensetzung nach Anspruch 1 durch Auflösung eines Lavendustinderivats der Formel I gemäß Definition von Anspruch 1 oder eines pharmazeutisch akzeptablen Salzes hiervon, in einem Emolliens und optional weiteren lipophilen Komponenten, wie Konsistenzmitteln, falls vorhanden, vorzugsweise bei erhöhter Temperatur, beispielsweise bei etwa 60 bis 80°C, und Zusatz der Wasserphase, falls vorhanden, unter Rührung und Homogenisation.

7. Zusammensetzung nach einem der Ansprüche 1 bis 4 zur Verwendung bei der Behandlung hyperproliferativer Störungen, wie aktinischer Keratose, anogenitaler Warzen und seborrhöischer Keratose und Hautkrebs.

8. Verwendung einer Zusammensetzung nach einem der Ansprüche 1 bis 4 bei der Herstellung eines Arzneimittels für die Behandlung hyperproliferativer Störungen, wie aktinischer Keratose, anogenitaler Warzen und seborrhöischer Keratose und Hautkrebs.

9. Verwendung nach Anspruch 8, worin eine Zusammensetzung nach einem der Ansprüche 1 bis 4 auf die Haut oder die Mukosa eines behandlungsbedürftigen Patienten verabreicht wird.

## Revendications

1. Composition pharmaceutique destinée à une administration par voie topique comprenant un dérivé de lavendustine de formule I 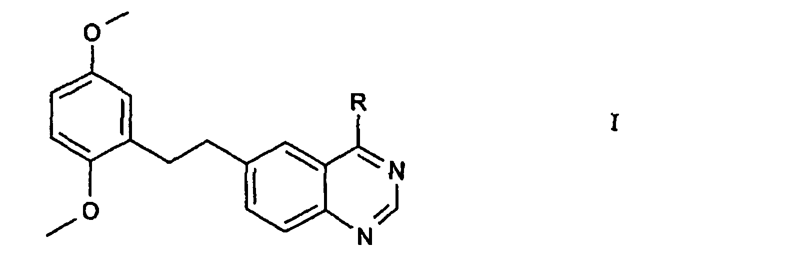 dans laquelle, R est méthyle, méthoxy ou éthyle, ou un de ses sels pharmaceutiquement acceptables, et un émollient.

2. Composition selon la revendication 1, **caractérisée en ce que** l'émollient est un myristate d'isopropyle.

3. Composition selon la revendication 1 se présentant sous forme d'une émulsion.

4. Composition selon la revendication 3 se présentant sous forme d'une émulsion huile dans l'eau.

5. Composition pharmaceutique destinée à une administration par voie topique selon l'une quelconque des revendications 1 à 4 qui permet d'éviter une importante concentration locale de lavendustine au niveau de la peau ou de la muqueuse et qui est bien tolérée.

6. Procédé pour la préparation d'une composition selon la revendication 1 qui consiste à dissoudre un dérivé de lavendustine de formule I tel que défini à la revendication 1, ou un de ses sels pharmaceutiquement acceptable, dans un émollinet ainsi qu'éventuellement d'autres composants lipophiles tels que des agents de consistance, si il y en a, de préférence à une température élevée, p.ex. d'environ 60 à environ 80°C, et à ajouter la phase aqueuse, si il y en a une, sous agitation ou homogénéisation.

7. Composition selon l'une quelconque des revendications 1 à 4 utilisable pour le traitement de troubles hyperprolifératifs tels que la kératose actinique, les verrues anogénitales et la kératose séborrhéique, ainsi que pour le traitement du cancer de la peau.

8. Utilisation d'une composition selon l'une quelconque des revendications 1 à 4 dans la préparation d'un médicament destiné au traitement de troubles hyperprolifératifs tels que la kératose actinique, les verrues anogénitales et la kératose séborrhéique, ainsi que du cancer de la peau.

9. Utilisation selon la revendication 8 **caractérisée en ce qu'**une composition selon l'une des revendications 1 à 4 doit être administrée au niveau de la peau ou de la muqueuse d'un patient le nécessitant.
